# EUROPEAN PATENT APPLICATION

(11) **EP 0 926 117 A1**
(43) Date of publication of application: **30.06.1999**
(21) Application number: 97310479.7
(22) Date of filing: 22.12.1997
(51) Int. Cl.: C07C 29/56, C07C 35/08

(54) **Preparation of isopulegol**

(71) Applicant: QUEST INTERNATIONAL B.V., 1411 GP Naarden (NL)
(72) Inventor: The designation of the inventor has not yet been filed
(74) Representative: Humphries, Martyn

(57) **Abstract**

Isopulegol is prepared by cyclisation of citronellal using scandium trifluoromethanesulphonate as catalyst.

## Description

### Field of the Invention

This invention concerns preparation of isopulegol.

### Background to the Invention

Isopulegol is a known fragrance material, with its most important use being as a precursor for menthol in a known hydrogenation reaction. Isopulegol is conventionally made by cyclisation of citronellal in the presence of zinc bromide catalyst, for example as described in Nakatani et al, Synthesis 1978, 147, although the zinc bromide is required in stoichiometric amounts as it forms a complex with the reaction products.

The present invention concerns an alternative approach to preparation of isopulegol.

### Summary of the Invention

According to the invention there is provided a method of preparing isopulegol by cyclisation of citronellal, characterised by use of scandium trifluoromethanesulphonate as catalyst.

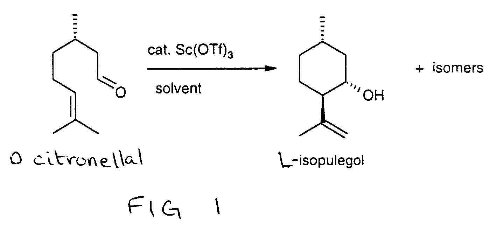

The reaction is illustrated in Figure 1.

Scandium trifluoromethanesulphonate (referred to for brevity as scandium triflate) functions as a true catalyst, and does not complex with the reaction products, giving good product yields when present in amounts of 5-10 mol%.

Isopulegol exists in a number of isomeric forms. For use as a precursor for production of menthol, it is desirable to have L-isopulegol (which has stereochemistry as shown in Figure 1), as this produces L-menthol, which is generally the most preferred form of menthol.

Use of scandium triflate as catalyst enables good selective production of L-isopulegol in preference to other isomers, by suitable selection of reaction conditions. This is desirable when the isopulegol is to be used as a precursor for production of menthol, for the reasons explained above.

Initial experiments have concentrated on finding a suitable set of conditions to give optimal yield and diastereomeric ratios.

Experiments have been carried out using different solvents, and these have shown that the choice of solvent has a significant effect: see results in Table 1.

**Table 1**

| Choice of solvent for the citronellal cyclisation reaction (0.2M aldehyde). | | | | |
|---|---|---|---|---|
| Solvent | mol% cat. | Time/hrs | Yield%* | Product ratio⁸ (L-isopulegol:others) |
| MeNO₂ | 5 | 1 | 50 | 78:22 |
| Et₂O | 5 | 1 | 38 | 84:16 |
| DCM | 5 | 2 | 58 | 80:20 |
| toluene | 5 | 2 | 45 | 82:18 |
| hexane | 5 | 2 | 13 | 87:13 |

| | | | | |
|---|---|---|---|---|
| * The remainder is high boiling (oligomeric) material | | | | |
| ⁸ The next most abundant isomer was neo-isopulegol (OH inverted compared with L-isopulegol as shown in Figure 1). Other isomers were only present in trace amounts. | | | | |

Et₂O is diethylether and DCM is dichloromethane.

From Table 1 it is evident that whilst polar solvents gave the better yields, they also showed lower selectivities. Conversely, non-polar solvents gave high selectivities but low yields.

Experiments were also carried out at different reaction temperatures, and it was found that lowering the reaction temperature gave increased selectivities: see results in Table 2.

**Table 2**

| Effect of temperature on yield and isomer ratios (0.1M aldehyde). | | | | |
|---|---|---|---|---|
| Temp/°C | mol%cat. | Time/hrs | Yield | Isomer ratio (L-isopulegol:others) |
| 25 | 5 | 2 | 58 | 80:20 |
| 0 | 5 | 0.5 | 45 | 81:19 |
| -40 | 10 | 0.5 | 86 | 88:12 |
| -78 | 10 | 1 | 100 | 94:6 |

It is interesting to note that both yields and diastereomer ratios improved on cooling. Whilst these reactions were carried out at low concentration, this was found unnecessary. At higher concentrations, reaction was also seen to occur smoothly: see results in Table 3.

**Table 3**

| Effect of increased concentration (1.0M aldehyde) and catalyst loading on yield and isomer ratio. | | | | |
|---|---|---|---|---|
| Temp/°C | mol%cat. | Time/hrs | Yield | Isomer ratio (L-isopulegol:others) |
| -78 | 10 | 0.75 | 100 | 94:6 |
| -78 | 5 | 1.5 | 100 | 94:6 |

It can be seen that the reaction occurs more rapidly at the higher concentration, and that there is no observed loss of diastereoselection. A lower catalyst loading is also shown to be practicable.

The present invention can thus provide an efficient catalytic route to production of L-isopulegol, giving the product in high diastereomeric excess (with results at least as good as those obtained using zinc bromide), and excellent yield (far exceeding that obtainable with zinc bromide).

The invention also covers isopulegol obtained by the method of the invention.

The resulting isopulegol preferably comprises L-isopulegol as the major product, typically in an amount of at least 80%, and desirably at least 90%.

Experiments so far have demonstrated the effectiveness in principle of use of sodium triflate as catalyst in production of isopulegol. The reaction has not yet been fully explored, and there is scope for further optimisation, eg to improve selectivity, catalyst loadings etc.

The invention also covers use of the isopulegol so produced for production of menthol, and the menthol so produced, particularly L-menthol.

The isopulegol is typically processed before production of menthol to produce 100% L-isopulegol, eg. by known distillation techniques.

## Claims

1. A method of preparing isopulegol by cyclisation of citronellal, characterised by use of scandium trifluoromethanesulphonate as catalyst.

2. A method according to claim 1, whereon citronellal and scandium trifluoromethanesulphonate are reacted at a temperature not exceeding 0°C.

3. A method according to claim 2, wherein the citronellal and scandium trifluoromethanesulphonate are reacted at a temperature not exceeding -40°C.

4. A method according to claim 3, wherein the citronellal and scandium trifluoromethanesulphonate are reacted at a temperature not exceeding -78°C.

5. A method according to any one of the preceding claims, using the solvent dichloromethane.

6. A method according to any one of the preceding claims, wherein the scandium trifluoromethanesulphonate is present in a molar amount in the range 5 to 10%.

7. Isopulegol produced by the method of any one of the preceding claims.

8. Isopulegol according to claim 7, comprising at least 80% L-isopulegol.

9. Isopulegol according to claim 8, comprising at least 90% L-isopulegol.

10. Menthol produced from isopulegol according to claim 7, 8 or 9.
